# EUROPÄISCHE PATENTANMELDUNG

(11) **EP 2 156 861 A1**
(43) Veröffentlichungstag der Anmeldung: **24.02.2010**
(21) Anmeldenummer: 09010630.3
(22) Anmeldetag: 18.08.2009
(51) Int. Cl.: A61N 5/06

(54) **Vorrichtung zur Erfassung und/oder Steuerung der den menschlichen Hormon-, insbesondere Serotonin-Haushalt beeinflussenden Lichtmenge**

(30) Priorität: 21.08.2008 DE 102008039124
(71) Anmelder: Bartenbach, Christian, 6071 Aldrans (AT)
(72) Erfinder: Bartenbach, Christian, 6071 Aldrans (AT)
(74) Vertreter: Thoma, Michael

(57) **Zusammenfassung**

Die vorliegende Erfindung betrifft eine Vorrichtung zur Erfassung und/oder Steuerung der lichtabhängige physiologische Parameter wie den menschlichen Hormon-, insbesondere Serotonin-Haushalt beeinflussenden Lichtmenge, mit einem am Körper tragbaren Messgerät (1), das einen Lichtmengenmesser (4) zur Messung der über die Zeit einwirkenden Menge des den Hormonspiegel beeinflussenden Lichts und eine Anzeigevorrichtung (5) zur Abgabe eines Lichtmangelsignals bei von einer Auswerteeinheit in Abhängigkeit der erfassten Lichtmenge bestimmten Lichtmangel aufweist. Erfindungsgemäß wird vorgeschlagen, dass das Messgerät einen Aktivitätssensor (2) zur Erfassung der physischen Aktivität des Messgeräteträgers und eine Zeiterfassungseinrichtung (5) zur Erfassung der zeitlichen Abhängigkeit der erfassten physischen Aktivität von der erfassten Lichtmenge aufweist, wobei eine Steuervorrichtung zur Steuerung der Abgabe des Lichtmangelsignals in Abhängigkeit der erfassten physischen Aktivität und deren zeitlicher Abhängigkeit von der erfassten Lichtmenge vorgesehen ist.

## Beschreibung

Die vorliegende Erfindung betrifft eine Vorrichtung zur Erfassung und/oder Steuerung der lichtabhängige physiologische Parameter wie den menschlichen Hormon-, insbesondere Serotonin-Haushalt beeinflussenden Lichtmenge.

Es ist seit geraumer Zeit bekannt, dass die für das Auge sichtbare bzw. vom Auge rezipierbare Lichtmenge den Serotoninspiegel des menschlichen Körpers beeinflusst und damit starke Auswirkungen auf das allgemeine Wohlbefinden, die Leistungsfähigkeit und den Schlafrhythmus des Menschen hat. Die einwirkende Tageslichtdosis beeinflusst dabei nicht nur den Hormonhaushalt, bei dem insbesondere die Schlaf- und Wachhormone Serotonin und Melotonin sowie das körpereigene Hormon Cortisol lichtabhängig sind, sondern auch weitere physiologische Parameter des autonomen Nervensystems wie beispielsweise die Atmung, den Blutdruck, die Verdauung oder die Herzfrequenz, die sich bei nicht ausreichender Tageslichtexposition verschlechtern. Zu den psychischen und physischen Auswirkungen von Lichtmangel gehören Erscheinungen wie Depressionen beispielsweise in Form der sog. Winterdepression, dauernde Abgespanntheit auch am Tage sowie Schlafstörungen in der Nacht bzw. Schlafmangel oder erhöhte Ruhepuls- und Herzschlagfrequenzen. Derartige Erscheinungen treten bevorzugt in der lichtarmen Jahreszeit auf und treffen insbesondere Menschen, die ihren Arbeitstag in geschlossenen Gebäuden verbringen, in die der Tageslichteinfall nur begrenzt ist. Ohne dies zu wissen, werden diese Auswirkungen bisweilen noch dadurch verschlimmert, dass beispielsweise zur Erzielung eines blendungsfreien Arbeitsplatzes Jalousien heruntergelassen werden oder das Gebäudebeleuchtungssystem bei vermeintlich hellen Tagen abgeschaltet wird, um Strom zu sparen.

Im Stand der Technik sind Lichtdosimeter bekannt, die nicht nur die jeweils aktuell herrschende Lichtintensität messen, sondern auch die über einen vorbestimmten Zeitraum auftretende Lichtmenge bestimmen. Maßgeblich für das psychologische und physiologische Wohlbefinden ist nämlich nicht eine kurzzeitige, hohe Lichtintensität, sondern eine ausreichend große Lichtmenge, d.h. eine ausreichende Luxzahl über eine ausreichende Zeitspanne. Luxstundenzähler sind beispielsweise aus der DD 238668 A1 oder der DE 103 50 053 B4 bekannt. Während die erstgenannte Schrift eine elektronische Kondensatorschaltung zur Bestimmung der Lichtdosis verwendet, setzt die letztgenannte Schrift hierzu ein chemisches Verfahren mit Indikatorpigmenten ein, die in Abhängigkeit der Lichtdosis einen Farbumschlag zeigen. Weiterhin ist aus der DE 10 2005 044 031 ein Lichttherapiegerät bekannt, dessen Spektralverteilung und Bestrahlungsstärke derart gesteuert wird, dass die Melatoninausschüttung verringert wird. Hierzu wird die über die Netzhaut des Auges aufnehmbare Lichtdosis gemessen, wobei die Trübung der Augenlinse berücksichtigt wird, um die Bestrahlungsquelle in ihrer Spektralverteilung und Bestrahlungsstärke anzupassen.

Derartige, vorbekannte Lichtdosimeter sind jedoch für den persönlichen Anwendungsbereich ungeeignet, da dem normalen Endverbraucher die angegebene Luxstundenzahl nicht wirklich weiterhilft, da die individuell benötigte Luxstundenzahl variieren kann und regelmäßig unbekannt ist. Zum anderen weicht die tatsächlich auf den menschlichen Organismus einwirkende Lichtdosis von der Lichtdosis ab, die mit am Arbeitsplatz bzw. in verschiedenen Räumen installierten Lichtdosimetern gemessen wird, insbesondere dann, wenn die jeweilige Person oft ihren Platz wechselt.

Darüber hinaus hängt die Wirksamkeit der Lichtdosis oft von anderen Umwelteinflüssen und/oder physiologischen Reaktionen ab, die von herkömmlichen Lichtdosimetern bzw. Luxstundenzählern nicht erfasst werden können.

Der vorliegenden Erfindung liegt die Aufgabe zugrunde, eine verbesserte Vorrichtung der genannten Art zu schaffen, die Nachteile des Standes der Technik vermeidet und Letzteren in vorteilhafter Weise weiterbildet. Vorzugsweise soll mit einfachen Mitteln die am menschlichen Organismus wirkende Lichtmenge präzise erfasst, die psychologische bzw. physiologische Bedeutung der erfassten Lichtmenge einfach erfassbar gemacht und die Auswertung der erfassten Lichtmenge an den individuellen "Lichthunger" des jeweiligen menschlichen Organismus angepasst werden.

Erfindungsgemäß wird die genannte Aufgabe durch eine Vorrichtung nach Anspruch 1 gelöst. Bevorzugte Ausgestaltungen der Erfindung sind Gegenstand der abhängigen Ansprüche.

Zur Erreichung der genannten Ziele schlägt die vorliegende Erfindung ein am Körper tragbares Messgerät vor, das nicht nur einen Lichtmengenmesser zur Messung der über die Zeit einwirkenden Lichtmenge des für die physiologischen Parameter relevanten Lichts, sondern auch einen Aktivitätssensor zur Erfassung der physischen Aktivität des Messgeräteträgers und Bestimmungsmittel zur Bestimmung des zeitlichen Zusammenhangs zwischen der erfassten physischen Aktivität und der erfassten Lichtmenge aufweist. Ein Lichtmangelsignal wird von einer Auswerteeinheit sowohl in Abhängigkeit der erfassten Lichtmenge als auch in Abhängigkeit der erfassten physischen Aktivität und deren zeitlicher Abhängigkeit von der erfassten Lichtmenge abgegeben. Die Berücksichtigung der physischen Aktivität und des zeitlichen Zusammenhangs des Auftretens dieser physischen Aktivität von der erfassten Lichtmenge erlaubt eine einfache Anpassung des Lichtmangelsignals an die individuell benötigte Luxstundenzahl und eine Berücksichtigung weiterer Einflussfaktoren auf die Wirksamkeit der Lichtdosis für den individuellen Probanden, da die Rückkopplung der gemessenen Lichtdosis mit Körperaktivitäten die Bestimmung der Wirksamkeit der einwirkenden Lichtmenge ermöglicht. Durch die Tragbarkeit am Körper wird immer die tatsächlich auf den menschlichen Organismus einwirkende Lichtmenge erfasst, wobei vorteilhafterweise nur Licht eines Wellenlängenbereichs erfaßt wird, das für die interessierenden lichtabhängigen physiologischen Parameter wie beispielsweise den das Wohlbefinden beeinflussenden Hormonspiegel und insbesondere den Serotoninspiegel tatsächlich relevant ist. Hierdurch können die Auswirkungen der erfassten Lichtdosis auf das psychische und/oder physische Wohlbefinden präziser erfasst werden. Vorteilhafterweise wird dabei von der Anzeigevorrichtung für den Träger leicht verständlich ein Lichtmangelsignal beispielsweise in Form einer optischen Anzeige "Lichtmangel" oder eines akustischen Anzeigesignals beispielsweise in der Form "Sie haben Lichtmangel" ausgegeben dann, wenn die erfasste Lichtdosis, die erfasste körperliche Aktivität und deren zeitliches Zusammenspiel von einer Auswerteeinheit dahingehend bewertet worden ist, dass der Träger des Messgeräts in einer vergangenen, vorbestimmten Zeitspanne zu wenig Licht ausgesetzt war. Alternativ oder zusätzlich können auch andere Anzeigesignale beispielsweise in Form eines Vibrationsalarms oder dergleichen Verwendung finden, um auf Lichtmangel hinzuweisen.

Um eine individuelle Anpassung der Lichtmangelbestimmung an den individuellen "Lichthunger" bzw. Bedarf des jeweiligen Trägers zu erreichen, ist vorgesehen, dass das Messgerät zusätzlich zur Messung der Lichtdosis mindestens einen physiologischen Parameter des Messgeräteträgers erfasst und/oder überwacht, der üblicherweise vom Lichtmangel beeinflusst wird und/oder von dem auf das Vorliegen von Lichtmangel rückgeschlossen werden kann. Grundsätzlich eignen sich hierfür verschiedene physiologische Größen wie beispielsweise Herzfrequenz, Lidschlagrate, Atemfrequenz, Blutdruck und dergleichen. Vorteilhafterweise berücksichtigt eine Steuervorrichtung, die die Abgabe des Lichtmangelsignals steuert, sowohl die vom Lichtmengenmesser über die Zeit erfasste Lichtmenge als auch den genannten zusätzlichen physiologischen Parameter des Messgeräteträgers.

Insbesondere besitzt das Messgerät einen Aktivitätssensor zur Erfassung der physiologischen Aktivität des Messgeräteträgers sowie eine Zeiterfassungseinheit, mittels derer die zeitliche Abhängigkeit der erfassten Aktivität von der erfassten Lichtmenge bestimmt wird. Üblicherweise wirkt sich die über das Auge rezipierte Lichtmenge zeitversetzt auf die körperliche Aktiviät aus insbesondere dergestalt, dass eine während des Tages aufgenommene ausreichende Tageslichtdosis nachts zu einem ruhigen Schlaf und dementsprechend zu einer verringerten Körperaktivität führt. Wird die Körperaktivität und deren zeitliche Abhängigkeit von der erfassten Lichtmenge analysiert, kann rückgeschlossen werden, ob die erfasste Lichtmenge für den individuellen Messgeräteträger ausreichend war oder nicht.

Der Aktivitätssensor kann hierbei grundsätzlich verschieden ausgebildet sein und prinzipiell jeden der zuvor genannten physiologischen Parameter erfassen. In vorteilhafter Weiterbildung der Erfindung kann der Aktivitätssensor einen Beschleunigungssensor und/oder einen Pulsmesser umfassen, um Körperbewegungen und deren Beschleunigung sowie die damit einhergehende Pulsfrequenz zu messen. Hieraus kann präzise die körperliche Aktivität charakterisiert werden, die ein Maß für den Aktivitätszustand des Körpers über einen vorbestimmten Zeitraum ist. Vorteilhafterweise können beispielsweise die Anzahl der auftretenden Beschleunigungen und/oder die Summe der Beschleunigungsbeträge über einen vorbestimmten Zeitraum erfasst werden. Alternativ oder zusätzlich können ein gewisses Maß übersteigende Abweichungen der Pulsfrequenz von einem Ruhepuls hinsichtlich der Anzahl der auftretenden Abweichungen und/oder der Summe der Abweichungsbeträge erfasst und mit entsprechenden Referenzwerten verglichen werden.

Die Bestimmung des Vorliegens von Lichtmangel auf Basis der ermittelten Lichtmenge und der ermittelten physiologischen Aktivität kann grundsätzlich in verschiedener Art und Weise vorgenommen werden. Nach einer vorteilhaften Ausführung der Erfindung kann Lichtmangel durch Vergleich der erfassten Größen mit Referenzwerten bestimmt werden. Hierzu kann die Steuervorrichtung einen Referenzwertvergleicher besitzen, der eine über einen bestimmten ersten Zeitraum erfasste Lichtmenge mit einem Lichtreferenzwert vergleicht und die über einen bestimmten zweiten, zum genannten ersten vergleichsweise späteren Zeitraum erfasste physiologische Aktivität mit einem Aktivitätsreferenzwert vergleicht. Ein Signalgeber der Steuervorrichtung gibt das zuvor genannten Lichtmangelsignal insbesondere dann ab, wenn die verglichene Lichtmenge den zugehörigen Lichtreferenzwert unterschreitet und/oder die verglichene physiologische Aktivität den Aktivitätsreferenzwert überschreitet.

Der genannte erste Zeitraum kann hierbei vorteilhafterweise ein vorbestimmtes Zeitfenster tagsüber sein, während der genannte zweite Zeitraum ein vorbestimmtes Zeitfenster nachtsüber sein kann. Die genannten Zeitfenster müssen hierbei nicht zwangsweise gleich lang sein und müssen nicht den gesamten Tag, d.h. 24 Stunden abdecken. Beispielsweise kann der genannte erste Zeitraum aus 2 Stunden am Vormittag und 2 Stunden am Nachmittag zusammengesetzt sein, während der genannten zweite Zeitraum 3 Stunden nach Mitternacht, beispielsweise von 1 Uhr nachts bis 4 Uhr nachts umfasst. Die genannten Zeiträume können jedoch individuell an den Arbeits- und Tagesrhythmus eines jeweiligen Messgeräteträgers angepasst werden und mittels einer Eingabevorrichtung entsprechend vorgebbar sein.

Als grundsätzlich vorteilhaft erweist es sich, wenn eine Lichtmenge von zumindest 5000 lx für zumindest 1 h vorzugsweise zumindest 4 Mal pro Woche einwirkt bzw. als Referenzwert gefordert wird. Alternativ können 2500 lx für 2 h oder 10000 lx für ½ h vorgesehen sein.

Alternativ oder zusätzlich zu einem solchen Referenzwertabgleich kann das Vorliegen von Lichtmangel nach einer weiteren vorteilhaften Ausführung der Erfindung auch mittels Fuzzy Logic bestimmt werden. Die Steuervorrichtung umfasst hierzu einen Fuzzy Logic-Regler, der die Abgabe des Lichtmangelsignals mithilfe von Fuzzy Logic steuert. Wie an sich bei dem Einsatz von Fuzzy Logic bekannt, können diverse Kriterien, die an sich betrachtet unscharfe Kriterien sein können, in verschiedenster Weise miteinander verknüpft werden, um zu einer korrelierten Aussage über das Vorliegen von Lichtmangel anhand der überwachten Parameter zu kommen.

In Weiterbildung der Erfindung kann das Wellenlängenfenster, innerhalb dessen die Lichtmenge aufgezeichnet wird, verändert werden. Mittels einer Einstellvorrichtung bzw. einer geeigneten Eingabevorrichtung kann ein Ausschnitt des Spektrums vorgegeben werden, innerhalb dessen die Lichtdosis bestimmt wird, so dass die Lichtdosisbestimmung individuell an den jeweiligen Träger angepasst werden kann. Beispielsweise kann ein begrenztes Wellenlängenfenster am oberen Rand des sichtbaren Spektrums vorgegeben werden, um aufzuzeichnen, welcher Lichtmenge der jeweilige Träger am oberen Rand des sichtbaren Spektrums ausgesetzt war. Durch diese Verschiebbarkeit und/oder Veränderung der Breite des spektralen Wellenlängenfensters kann die Lichtmengenbestimmung gezielt an einen jeweiligen Träger und dessen Wohlbefindlichkeits- bzw. Unwohlbefindlichkeitsbild angepasst werden. Alternativ oder zusätzlich kann hierbei vorgesehen sein, dass mehrere Wellenlängenbereiche definierbar sind, innerhalb derer die Lichtdosis aufgezeichnet wird. Vorteilhafterweise kann das Vorgeben der Wellenlängenfenster mit dem Vorgeben der zuvor genannten Aufzeichnungszeiträume variabel verknüpft werden, so dass beispielsweise in einem ersten Zeitraum am Vormittag die Menge des Lichts am oberen Rand des sichtbaren Spektrums bestimmt wird, während in einem zweiten Zeitfenster am Nachmittag die Menge des Lichts in einem Frequenzbereich am unteren Rand des sichtbaren Bereichs ermittelt wird. Die Vorgebbarkeit verschiedener Wellenlängenfenster und Zeiträume für die Aufzeichnung ermöglicht eine beträchtlich feinere Anpassung an individuelle Gegebenheiten und eine feinere Steuerung des Serotoninhaushalts.

Vorteilhafterweise ist hierbei der Lichtmengenmesser derart ausgebildet, dass er zwischen Tageslicht und Kunstlicht differenzieren kann. Tageslicht zeichnet sich gegenüber Kunstlicht in der Regel dadurch aus, dass es UV-Licht-Anteile und/oder Infrarotstrahlungsanteile enthält. Vorteilhafterweise ist daher der Lichtmengenmesser mit einem elektromagnetischen Strahlungssensor versehen, der über den sichtbaren Bereich hinaus elektromagnetische Strahlung erfassen kann, insbesondere Infrarotstrahlung und/oder UV-Licht erfassen kann. Wird von einer mit dem Strahlungssensor verbundenen Auswerteeinheit anhand eines Vorliegens von Infrarotstrahlung, UV-Licht und/oder einer Analyse des erfassten Strahlungsspektrums festgestellt, dass es sich um Tageslicht handelt, wird die entsprechende, auf den Lichtmengenmesser einwirkende Lichtmenge aufsummiert bzw. berücksichtigt. Auch wenn vorteilhafterweise in der genannten Weise der Strahlungssensor über den sichtbaren Bereich hinaus elektromagnetische Strahlung erfasst, kann in der vorgenannten Weise ein Wellenlängenfenster festgelegt werden, innerhalb dessen die Lichtmenge zur Bestimmung der Lichtdosis aufsummiert wird. Beispielsweise kann also das Vorliegen von UV-Licht bestimmt werden, um das Licht als Tageslicht zu identifizieren, dann aber das genannte UV-Licht nicht berücksichtigt werden, um die relevante Lichtdosis zu bestimmen. Insbesondere kann das Wellenlängenfenster so festgelegt sein, dass nur die Dosis des für das menschliche Auge sichbaren Lichts gemessen bzw. bestimmt wird.

Um eine für den jeweiligen Messgeräteträger passende Aussage über das Vorliegen von Lichtmangel zu erhalten, wird die Auswertung der erfassten Lichtmenge und/oder des zusätzlich erfassten physischen Parameters, insbesondere der erfassten physiologischen Aktivität dynamisch an den individuellen Lichthunger des jeweiligen Messgeräteträgers angepasst. Insbesondere kann hierzu die Steuervorrichtung einen adaptiven und/oder dynamischen Regler umfassen, der für den zuvor genannten Referenzwertabgleich den Lichtreferenzwert erhöht, wenn die im genannten ersten Zeitraum erfasste Lichtmenge über dem bis dato geltenden Lichtreferenzwert lag und die erfasste Aktivität im genannten zweiten Zeitraum über dem Aktivitätsreferenzwert lag. Durch diese dynamische Anpassung des Lichtreferenzwertes wird der Erscheinung Rechnung getragen, dass eine an sich ausreichende Lichtmenge am Tage an sich ein Absenken der physiologischen Aktivität bei Nacht zur Folge haben müsste. Wird jedoch bei an sich ausreichender Lichtmenge festgestellt, dass nachts trotzdem eine erhöhte körperliche Aktivität stattfindet, z.B. in Folge unruhigen Schlafes, Schlafmangels etc., kann rückgeschlossen werden, dass die individuell erhaltene Lichtdosis am Tag nicht ausreichend war. Umgekehrt kann der Lichtreferenzwert abgesenkt werden, wenn auch bei Unterschreiten des zuvor geltenden Lichtreferenzwertes nachts eine ausreichend abgesenkte Körperaktivität festgestellt wird. Durch eine solche dynamische Anpassung der Auswertung der erfassten Lichtmenge und des zumindest einen zusätzlich erfassten physiologischen Parameters aneinander kann die Ausgabe des Lichtmangelsignals an den individuellen Lichthunger eines Messgeräteträgers und dessen Schwankungen über längere Zeiträume wie beispielsweise Jahreszeiten bestens angepasst werden, so dass Fehlmeldungen besser vermieden werden können.

Die Bestimmung von Lichtmangel muss dabei nicht unmittelbar aus der bestimmten Lichtmenge selbst ermittelt werden, sondern kann auch aus einer daraus abgeleiteten Größe bestimmt werden. Insbesondere kann es vorteilhaft sein, aus einer bestimmte Lichtmenge zunächst die damit einhergehende Serotoninausschüttungsmenge - oder ggf. die Ausschüttungsmenge eines anderen Hormons - zu bestimmen und die solchermaßen für mehrere Zeiträume individuell bestimmten Serotoninausschüttungsmengen über einen vorgebbaren längeren Zeitraum aufzusummieren, die aufsummierte Serotoninausschüttungsmenge mit einem Referenzwert zu vergleichen und aus diesem Vergleich das Vorliegen von Lichtmangel zu bestimmen. Hierdurch können nicht lineare Zusammenhänge zwischen Lichtdosis und Serotoninausschüttung präziser erfasst werden, beispielsweise kann ein Schwellwerteffekt berücksichtigt und hieraus resultierende Ungenauigkeiten verringert werden. Würden über mehrere Zeiträume hinweg - beispielsweise täglich - geringe Lichtmengen aufsummiert, die jeweils für sich betrachtet noch keine signifikante Serotoninausschüttung bewirken, käme in der Summe über einen längeren Zeitraum - beispielsweis eine Woche - doch eine signifikante Lichtmenge zustande, die sich allerdings nicht in einer entsprechenden Serotoninausschüttungsmenge wiederspiegelt. Andere Effekte können hierdurch ebenfalls Berücksichtigung finden. Vorteilhafterweise kann beispielsweise die genannte Serotoninausschüttungsmenge jeweils über eine abgespeicherte Lichtmengen-/Serotoninfunktion bestimmt werden, anhand derer eine entsprechende Lichtmengen-Auswerteeinrichtung die jeweilige Serotoninausschüttungsmenge bestimmt, die sodann aufsummiert werden und alternativ oder zusätzlich zu den Lichtmengen selbst berücksichtigt werden können, um das Vorliegen von Lichtmangel zu bestimmen.

Nach einer vorteilhaften Ausführung der Erfindung kann das am Körper tragbare Messgerät vollständig autark arbeitend ausgebildet sein. Insbesondere kann die zuvor genannte Steuervorrichtung in das am Körper tragbare Messgerät integriert sein, so dass das Messgerät selbständig die erfassten Lichtmengendaten und die weiteren physiologischen Daten wie die Körperaktivität auswertet und in die Ausgabe des Lichtmangelsignals umsetzt.

Alternativ oder zusätzlich kann das genannte, am Körper tragbare Messgerät eine vorzugsweise berührungslos arbeitende Datenübertragungsvorrichtung besitzen, mittels derer die erfassten Daten betreffend Lichtmenge und/oder physische Aktivität auf ein externes Auswerte- und/oder Steuersystem übertragen kann. Mithilfe dieser Datenübertragung können vielerlei externe, zusätzliche Auswertungen der erfassten Daten erfolgen. Beispielsweise kann am PC eines Arztes exakt die über längere Zeiträume rezipierte Lichtmenge und/oder deren Verlauf in zeitlicher Abhängigkeit von den zusätzlichen physiologischen Daten ausgewertet werden. Alternativ oder zusätzlich zu einer solchen berührungslos arbeitenden Datenübertragungsvorrichtung kann das Messgerät auch einen herausnehmbaren Datenspeicher beispielsweise in Form eines Chips umfassen, der in ein externes Auswerte- und/oder Steuersystem eingesetzt und/oder eingelesen werden kann.

Insbesondere kann die Datenübertragung vom am Körper tragbaren Messgerät jedoch dazu genutzt werden, externe Geräte, die das psychische und/oder physische Wohlbefinden des Messgeräteträgers beeinflussen, in Abhängigkeit der erfassten Lichtmenge und/oder des erfassten zusätzlichen physiologischen Parameters zu steuern.

In besonders vorteilhafter Weiterbildung der Erfindung werden die durch das am Körper tragbare Messgerät erfassten Daten betreffend Lichtmenge und/oder körperliche Aktivität auf ein Gebäudetechnik-Steuergerät zur Steuerung einer Gebäudefunktion übertragen, das die genannte zumindest eine Gebäudefunktion in Abhängigkeit der übertragenen Daten betreffend Lichtmenge und/oder körperliche Aktivität und/oder hieraus abgeleiteter Daten steuert.

Vorteilhafterweise können in Abhängigkeit der übertragenen, erfassten Lichtmenge und erfassten körperlichen Aktivität solche Gebäudefunktionen gesteuert werden, die unmittelbar Einfluss auf die auf den Körper einwirkende serotoninrelevante Lichtmenge haben. In Weiterbildung der Erfindung kann das genannte Gebäudetechnik-Steuergerät, das mit dem am Körper tragbaren Messgerät kommuniziert, eine Verschattungsanlage wie beispielsweise Jalousien oder Rollos und/oder eine Gebäudelichtanlage in Abhängigkeit der am Messgerät erfassten Lichtmenge und/oder in Abhängigkeit der am Messgerät erfassten körperlichen Aktivität steuern.

Wird beispielsweise anhand der Daten, die das am Körper tragbare Messgerät erfasst, ein Lichtmangel festgestellt, kann das Gebäudetechnik-Steuergerät automatisch über einen vorbestimmten Zeitraum die Jalousien tagsüber öffnen, auch wenn dies beispielsweise einem reflexionsfreien Arbeitsplatz abträglich ist und/oder zur Kühlung des Gebäudes an sich anders vorgesehen wäre. Alternativ oder zusätzlich kann die Raumbeleuchtung eingeschaltet und/oder hinsichtlich der Luxzahl erhöht werden, wenn sich ein unter Lichtmangel leidender Messgeräteträger im Raum befindet.

Alternativ oder zusätzlich können auch weitere Gebäudefunktionen wie beispielsweise Klimaanlage, Heizung und dergleichen in Abhängigkeit der Daten gesteuert werden, die von dem am Körper tragbaren Messgerät auf das Gebäudetechnik-Steuergerät übertragen werden.

In vorteilhafter Weiterbildung der Erfindung kann in Abhängigkeit der vom Messgerät bestimmten Lichtmenge und/oder der bestimmten körperlichen Aktivität und/oder hieraus abgeleiteten Daten auch ein Therapiegerät, insbesondere ein Lichttherapiegerät beispielsweise in Form einer Lichttherapiekabine vorzugsweise automatisch angesteuert werden, deren Behandlungsparameter entsprechend eingestellt werden. Beispielsweise kann die ansonsten voreingestellte Lichtdosis erhöht oder die Bestrahlungsdauer verlängert werden, wenn die eingelesenen Daten einen größeren Lichtmangel implizieren.

In vorteilhafter Weiterbildung der Erfindung ist hierbei das am Körper tragbare Messgerät und/oder das damit kommunizierende Auswerte- und/oder Steuersystem derart ausgebildet, dass die Datenübertragung und/oder die aus den übertragenen Daten abgeleitete Funktionssteuerung automatisch bewerkstelligt wird. Dies kann beispielsweise durch eine sich automatisch intervallmäßig wiederholende Datenübertragung erfolgen.

Vorteilhafterweise jedoch arbeitet die Datenübertragungsvorrichtung des Messgeräts ortsabhängig insbesondere dergestalt, dass die am Messgerät erfassten und/oder abgeleiteten Daten automatisch beim Betreten eines Gebäudes und/oder beim Betreten eines Raumes auf das Gebäudetechnik-Steuergerät übertragen und/oder für dieses abrufbar bereitgestellt werden. In Weiterbildung der Erfindung kann das am Körper tragbare Messgerät einen Transponder und/oder ein RFID-Modul aufweisen, von dem die Daten betreffend Lichtmenge und körperliche Aktivität abrufbar bzw. bereitgestellt sind. Das Gebäudetechnik-Steuergerät kann vorteilhafterweise eine im Bereich einer Eingangstüre angeordnete Leseeinheit in Form eines RFID-Lesers und/oder eines Transponderchip-Lesers aufweisen, der dann, wenn die jeweilige das Messgerät tragende Person durch die Tür tritt, die Daten betreffend Lichtmangel und/oder körperliche Aktivität ausliest und zumindest eine der zuvor genannten Gebäudefunktionen automatisch in Abhängigkeit der ausgelesenen Daten steuert.

Der Lichtmengenmesser des am Körper tragbaren Messgeräts kann grundsätzlich verschieden ausgebildet sein. Um eine einfache Datenverarbeitung bei einer kompakten und kostengünstigen Ausbildung des Messgeräts zu ermöglichen, ist in vorteilhafter Ausführung der Erfindung ein optischer Sensor vorgesehen, der ein von der Lichtintensität abhängiges Lichtintensitätssignal abgibt. Um nicht nur die punktuell aktuelle Lichtintensität, sondern die über die Zeit wirkende Lichtmenge zu erfassen, kann der Lichtmengenmesser ein mit dem optischen Sensor verknüpften Summenbildungsbaustein beispielsweise in Form eines Integrators aufweisen, der die Lichtmenge über die Zeit bestimmt. Um die Lichtmengen in vorgebbaren Wellenlängenfenstern bestimmen zu können, kann dem optischen Sensor eine Wellenlängen-Auswahlvorrichtung vor- oder zugeordnet werden, um nur Licht eines bestimmten Wellenlängenbereichs zu erfassen.

Das am Körper tragbare Messgerät kann hierbei grundsätzlich verschieden ausgebildet sein, wobei es in jedem Fall vorteilhafteniveise ausreichend miniaturisiert ist, um permanent am Körper getragen werden zu können, ohne als störend empfunden zu werden. Dies ist eine wichtige Voraussetzung, damit das Messgerät tatsächlich permanent getragen wird, um eine tatsächlich akkurate Bestimmung der auf den Träger einwirkenden Lichtdosis zu ermöglichen. Das Messgerät bildet vorzugsweise ein Kleingerät des persönlichen Bedarfs, das elektronisch arbeitend ausgebildet sein kann.

In vorteilhafter Weiterbildung der Erfindung kann das Messgerät als um den Hals tragbares Amulett ausgebildet sein. Alternativ oder zusätzlich kann ein am Handgelenk tragbares Armbandgerät als Messgerät vorgesehen sein. Besonders vorteilhaft kann es auch sein, daß Meßgerät in eine Brille, insbesondere deren Gestell zu integrieren. Alternativ oder zusätzlich kann auch ein Meßgerät in Form einer Gürtelschnalle, eines Fingerrings, eines Ohrrings, einer ansteckbaren Brosche oder eines elastischen Bandes verwendet werden, das um den Arm oder das Bein gelegt werden kann.

Die Erfindung wird nachfolgend anhand eines bevorzugten Ausführungsbeispiels und zugehöriger Zeichnungen näher erläutert. In den Zeichnungen zeigen:
- Fig. 1:: eine schematische Darstellung eines als Armbandgerät ausgebildeten Messgeräts zur Messung der auf einen Messgeräteträger einwirkenden Lichtdosis,
- Fig. 2:: eine schematische Darstellung des am Körper tragbaren Messgeräts aus Fig. 1 und dessen Bausteine umfassend einen optischen Lichtintensitätssensor, einen Beschleunigungssensor, ein Anzeigedisplay, eine Steuer- und Auswerteeinheit sowie eine Datenübertragungsvorrichtung in Form eines RFID-Moduls, und
- Fig. 3:: eine schematische Darstellung der mit dem am Körper tragbaren Messgerät kommunizierenden Gebäudetechnik zur automatischen Steuerung von Gebäudefunktionen in Abhängigkeit der am Körper erfassten Lichtmenge.

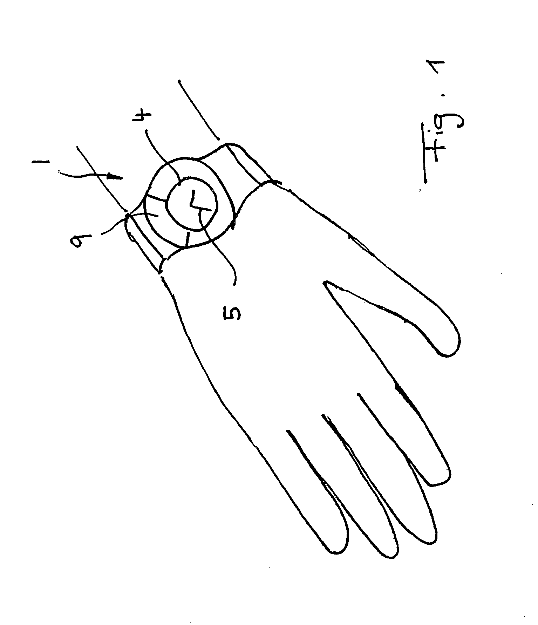

Wie Fig. 1 zeigt, kann das am Körper tragbare Messgerät 1, mittels dessen die auf einen Messgeräteträger einwirkende Lichtdosis, d.h. die Lichtmenge über die Zeit, sowie zusätzlich dessen Aktivität gemessen wird, als Armbandgerät nach Art einer Armbanduhr ausgebildet sein, die um das Handgelenk getragen werden kann. Das Messgerät 1 könnte alternativ jedoch auch in anderer Art und Weise am Körper getragen werden, insbesondere auch als um den Hals hängbares Amulett, oder auch in anderer Weise - wie bespielsweise als Brosche, als Gürtelschnalle, Arm- oder Fußband oder insbesondere auch als Brille - , solange sichergestellt ist, dass das Messgerät 1 dem Tageslicht ausgesetzt ist. Die in Fig. 1 dargestellte Ausführung als Armbandgerät besitzt den Vorteil, dass Körperbewegungen von dem Messgerät 1 unmittelbar und vollständig mitgemacht werden, wodurch die Körperaktivität sehr präzise messbar und überwachbar ist. Zudem erlaubt es das Anbringen am Handgelenk auch in einfacher Weise beispielsweise den Puls des Trägers zu überwachen.

In der gezeichneten Ausführung umfasst das Messgerät 1 als Aktivitätssensor 2 zur Erfassung der physischen Aktivität des Messgeräteträgers einen Beschleunigungssensor 3, der es erlaubt, Körperbewegungen hinsichtlich ihrer Beschleunigung und der daraus resultierenden Geschwindigkeit zu charakterisieren. Wie gesagt könnte jedoch auch ein zusätzliches physiologisches Merkmal, welches im Zusammenhang mit der vom Auge rezipierten Tageslichtmenge steht, überwacht werden, um die Körperaktivität infolge der Lichtmenge zu überwachen.

Zusätzlich zu dem genannten Aktivitätssensor 2 umfasst das Messgerät 1 einen Lichtmengenmesser 4, der über die Zeit die einwirkende Lichtmenge des für das menschliche Auge sichtbaren bzw. des serotoninspiegelrelevanten Lichts misst. Der Lichtmengenmesser 4 kann hierzu vorteilhafterweise einen optischen Sensor aufweisen, der auf Licht im Wellenlängenbereich von 380 nm bis 780 nm anspricht und ein die Lichtintensität im genannten Wellenlängenbereich angebendes Signal bereitstellt. Mittels eines vorteilhafterweise in das Messgerät 1 integrierten Summenbildungsbaustein kann das genannte Lichtintensitätssignal über die Zeit aufsummiert bzw. integriert werden, um die über die Zeit wirkende Lichtmenge beispielsweise in Luxstunden zu bestimmen. Der genannte Summenbildungsbaustein kann hierzu beispielsweise in Form eines Softwareprogramms in eine Datenverarbeitungseinrichtung mit Mikroprozessor programmiert sein, die in das Messgerät 1 eingebaut ist.

Vorteilhafterweise umfasst das Messgerät 1 darüber hinaus eine Zeiterfassungseinrichtung 5, die es ermöglicht, die vom Aktivitätssensor 2 bestimmte Körperaktivität in zeitlichem Zusammenhang zu der bestimmten Lichtmenge zu setzen. Insbesondere kann die in einem vorzugsweise nachts liegenden zweiten Zeitfenster erfasste Körperaktvität in Beziehung gesetzt werden zu der während eines vorzugsweise tagsüber liegenden ersten Zeitfensters erfassten Lichtmenge. Dies kann in der eingangs beschriebenen Art und Weise mittels eines Fuzzy Logic-Reglers und/oder über Vergleich mit vorbestimmten Referenzwerten und deren dynamische Anpassung erfolgen.

Bestimmt die Auswerteeinheit 7 der Datenverarbeitungseinrichtung 6 des Messgeräts 1, die vorteilhafterweise ebenfalls in Form eines Softwareprogramms realisiert sein kann, dass ein Lichtmangel vorliegt, der beispielsweise Schlafmangel bzw. Schlafstörungen nach sich zieht, die Gefahr von Depressionen birgt oder sich allgemein in Niedergeschlagenheit bzw. begrenzter Leistungsfähigkeit zeigen kann, dann gibt die Steuervorrichtung 8, mittels derer eine am Messgerät 1 vorgesehene Anzeigevorrichtung 9 beispielsweise in Form eines Anzeigedisplays angesteuert wird, auf der genannten Anzeigevorrichtung 9 ein Lichtmangelsignal beispielsweise in Form einer grafisch dargestellten Meldung "Lichtmangel" aus, die den Messgeräteträger darüber informiert, dass er sich in stärkerem Maße dem Tageslicht aussetzen sollte.

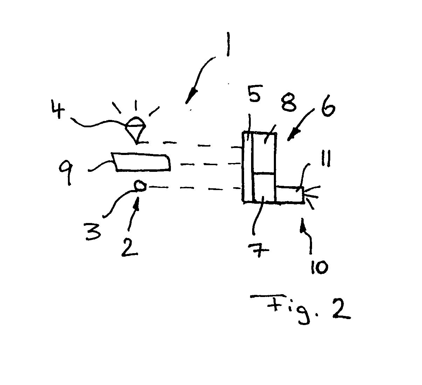

Wie Fig. 2 zeigt, ist die Datenverarbeitungseinrichtung 6 des Messgeräts 1 mit einer Datenschnittstelle 10 versehen, die es erlaubt, die vom Messgerät 1 gesammelten und/oder ausgewerteten Daten betreffend die Lichtmenge und die körperliche Aktivität auszulesen. Vorteilhafterweise kann die Datenschnittstelle 10 hierzu eine drahtlos arbeitende Datenübertragungsvorrichtung 11 umfassen, die vorteilhafterweise in einen Transponderchip bzw. RFID-Tag integriert sein bzw. von einem solchen gebildet sein kann.

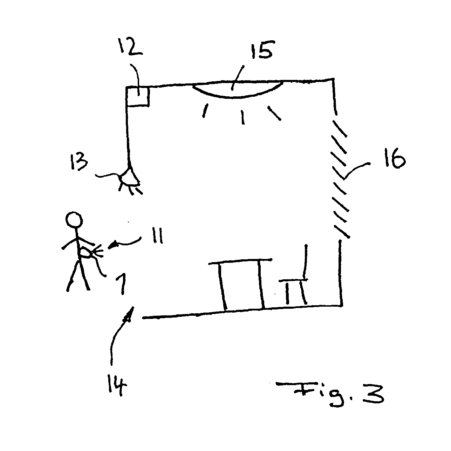

Durch die genannten Datenübertragungsvorrichtung 11 wird es vorteilhafterweise ermöglicht, die von dem Messgerät 1 gesammelten und/oder ausgewerteten Daten betreffend die Lichtmenge und die körperliche Aktivität bzw. ggf. weitere physiologische Parameter auf ein externes Auswerte- und/oder Steuersystem vorzugsweise automatisch zu übertragen. Insbesondere kann es hierbei vorteilhaft sein, die genannten Daten des Messgeräts 1 auf ein Gebäudetechnik-Steuergerät 12 zu übertragen, wie dies Fig. 3 verdeutlicht. Hierzu kann das genannte Gebäudetechnik-Steuergerät 12 in vorteilhafter Weise mit einem Datenlesegerät 13 versehen sein, das im Bereich einer Eingangstür des Gebäudes bzw. einer Eingangstür eines jeweiligen Raumes angeordnet ist und sozusagen im Vorbeigehen die Daten des Messgeräts 1 ausliest, die in dessen Transponderchip bzw. RFID-Tag gespeichert sind, wenn ein jeweiliger Messgeräteträger durch den genannten Eingang 14 das Gebäude bzw. den Raum betritt.

In der in Fig. 3 schematisch dargestellten Ausführungsform wird von dem genannten Gebäudetechnik-Steuergerät 12 einerseits eine Lichtanlage 15 sowie eine Verschattungsanlage 16 beispielsweise in Form einer Fensterjalousie gesteuert. Alternativ oder zusätzlich zur Lichtanlage 15 und zur Verschattungsanlage 16 kann das Gebäudetechnik-Steuergerät 12 ggf. auch andere Gebäudefunktionen in Abhängigkeit der Daten des Messgeräts 1 ansteuern, beispielsweise eine Klimaanlage, eine Heizung oder auch speziellere Einrichtungen wie beispielsweise eine Lichttherapiekabine, in der eine jeweilige Person gezielt mit serotoninspiegelrelevantem Licht beaufschlagt werden kann.

Betritt der in Fig. 3 dargestellte Messgeräteträger zusammen mit dem Messgerät 1 den Raum durch den Eingang 14, werden automatisch die im Messgerät 1 bestimmten, ausgewerteten und/oder gespeicherten Daten betreffend die Lichtmenge und die Körperaktivität vom Datenlesegerät 13 eingelesen und auf das Gebäudetechnik-Steuergerät 12 übertragen. Wurde beispielsweise ein Lichtmangel festgestellt, kann das genannte Gebäudetechnik-Steuergerät 12 beispielsweise die Lichtanlage 15 automatisch einschalten und/oder hinsichtlich der hiervon erzeugten Lichtintensität höher stellen oder die Verschattungsanlage 16 automatisch öffnen bzw. zumindest heller stellen, auch wenn dies an sich aufgrund der Tageslichtverhältnisse sonst nicht vorgesehen wäre.

## Patentansprüche

1. Vorrichtung zur Erfassung und/oder Steuerung der lichtabhängige physiologische Parameter, vorzugsweise den menschlichen Hormon-, insbesondere Serotoninhaushalt, beeinflussenden Lichtmenge, mit einem am Körper tragbaren Messgerät (1), das einen Lichtmengenmesser (4) zur Messung der über die Zeit einwirkenden Menge des den physiologischen Parameter, vorzugsweise Hormonspiegel beeinflussenden Lichts und eine Anzeigevorrichtung (9) zur Abgabe eines Lichtmangelsignals bei von einer Auswerteeinheit in Abhängigkeit der erfassten Lichtmenge bestimmtem Lichtmangel aufweist, **dadurch gekennzeichnet, dass** das Messgerät (1) einen Aktivitätssensor (2) zur Erfassung der physischen Aktivität des Messgeräteträgers und eine Zeiterfassungseinrichtung (5) zur Erfassung der zeitlichen Abhängigkeit der erfassten physischen Aktivität von der erfassten Lichtmenge aufweist, wobei eine Steuervorrichtung (8) zur Steuerung der Abgabe des Lichtmangelsignals in Abhängigkeit der erfassten physischen Aktivität und deren zeitlicher Abhängigkeit von der erfassten Lichtmenge vorgesehen ist.

2. Vorrichtung nach dem vorhergehenden Anspruch, wobei die Steuervorrichtung (8) einen Referenzwertvergleicher, der eine über einen bestimmten ersten, vorzugsweise tagsüber liegenden Zeitraum erfasste Lichtmenge mit einem Lichtreferenzwert vergleicht und die über einen bestimmten zweiten, vergleichsweise späteren, vorzugsweise nachtsüber liegenden Zeitraum erfasste physische Aktivität mit einem Aktivitätsreferenzwert vergleicht, und einen Signalgeber, der das Lichtmangelsignal dann abgibt, wenn die verglichene Lichtmenge ihren Lichtreferenzwert unterschreitet und/oder die verglichene Aktivität ihren Aktivitätsreferenzwert überschreitet, aufweist.

3. Vorrichtung nach dem vorhergehenden Anspruch, wobei die Steuervorrichtung (8) einen adaptiven Regler aufweist, der den Lichtreferenzwert erhöht, wenn die im bestimmten ersten Zeitraum erfasste Lichtmenge über dem für den Vergleich herangezogenen Lichtreferenzwert lag und die erfasste Aktivität im genannten zweiten Zeitraum über dem Aktivitätsreferenzwert lag, und/oder den Lichtreferenzwert erniedrigt, wenn die im bestimmten ersten Zeitraum erfasste Lichtmenge unter dem für den Vergleich herangezogenen Lichtreferenzwert lag und die erfasste Aktivität im genannten zweiten Zeitraum unter dem Aktivitätsreferenzwert lag.

4. Vorrichtung nach einem der vorhergehenden Ansprüche, wobei ein Fuzzy Logic-Regler zur Regelung der Abgabe des Lichtmangelsignals mithilfe von Fuzzy Logic vorgesehen ist.

5. Vorrichtung nach einem der vorhergehenden Ansprüche, wobei eine Lichtmengen-Auswerteeinrichtung vorgesehen ist, die in Abhängigkeit der über mehrere Zeiträume hinweg erfassten Lichtmengen jeweils eine Serotoninausschüttungsmenge vorzugsweise anhand einer gespeicherten Lichtmengen-/Serotoninfunktion bestimmt, die über einen vorgebbaren Zeitraum bestimmten Serotoninausschüttungsmengen aufsummiert und die aufsummierte Serotoninausschüttungsmenge mit einem Serotoninreferenzwert vergleicht, und wobei ein Signalgeber, der das Lichtmangelsignal dann abgibt, wenn die aufsummierte Serotoninausschüttungsmenge unter ihrem Referenzwert liegt, vorgesehen ist.

6. Vorrichtung nach einem der vorhergehenden Ansprüche, wobei das Messgerät (1) eine Datenübertragsvorrichtung (11), vorzugsweise einen Transponderchip und/oder ein RFID-Modul, zur Übertragung der erfassten Daten betreffend Lichtmenge und/oder Aktivität und/oder aus den erfassten Daten abgeleiteten Daten auf ein externes Auswerte- und/oder Steuersystem aufweist.

7. Vorrichtung nach dem vorhergehenden Anspruch, wobei das externe Auswerte- und Steuersystem ein Gebäudetechnik-Steuergerät (12) zur Steuerung einer Gebäudefunktion in Abhängigkeit der übertragenen Daten betreffend Lichtmenge und/oder körperliche Aktivität von dem am Körper tragbaren Messgerät (1) aufweist, wobei vorzugsweise das Gebäudetechnik-Steuergerät (12) Steuermittel zur Steuerung einer Verschattungsanlage (16) und/oder Steuermittel zur Steuerung einer Lichtanlage (15) des Gebäudes in Abhängigkeit der am Messgerät (1) erfassten Lichtmenge und/oder ein im Bereich eines Eingangs (14) angeordnetes Datenlesegerät (13) zum automatischen Auslesen der Daten des Messgeräts (1) beim Betreten des Gebäudes und/oder eines Raumes des Gebäudes aufweist.

8. Vorrichtung nach Anspruch 6 oder 7, wobei das externe Auswerte- und/oder Steuersystem ein Therapiegeräte-Steuergerät zur Steuerung eines Lichtherapiegeräts in Abhängigkeit der übertragenen Daten betreffend Lichtmenge und/oder körperliche Aktivität von dem am Körper tragbaren Messgerät (1) und/oder hieraus abgeleiteter Daten aufweist.

9. Vorrichtung nach einem der vorhergehenden Ansprüche, wobei der Lichtmengenmesser (4) einen optischen Sensor zur Abgabe eines von der Lichtintensität abhängigen Lichtintensitätssignals sowie einen Summenbildungsbaustein zur Bestimmung der Lichtmenge über die Zeit aufweist.

10. Vorrichtung nach einem der vorhergehenden Ansprüche, wobei eine Einstellvorrichtung zur variablen Einstellung des Zeitfensters, innerhalb dessen die Lichtmenge erfaßt wird, und/oder des Zeitfensters, innerhalb dessen die körperliche Aktivität erfaßt wird, und/oder eine Einstellvorrichtung zur variablen Einstellung des Wellenlängenfensters, innerhalb dessen die Lichtmenge bestimmt wird, vorgesehen ist.

11. Vorrichtung nach einem der vorhergehenden Ansprüche, wobei der Lichtmengenmesser (4) Bestimmungsmittel zur Bestimmung, ob Tageslicht oder Kunstlicht auf den Lichtmengenmesser (4) einwirkt, aufweist und einen Tageslicht-Summenbildungsbaustein zur Bestimmung der Tageslichtmenge über die Zeit aufweist, wobei die Steuervorrichtung für die Abgabe des Lichtmangelsignals das Tageslicht berücksichtigt und das Kunstlicht unberücksichtigt lässt.

12. Vorrichtung nach dem vorhergehenden Anspruch, wobei die Bestimmungsmittel zumindest einen elektromagnetischen Strahlungssensor zur Erfassung von Infrarotstrahlung und/oder ultravioletter Strahlung aufweisen und der Tageslicht-Summenbildungsbaustein in Abhängigkeit eines Signals des elektromagnetischen Strahlungssensors aktiviert wird derart, dass nur bei Vorliegen eines Infrarotanteils und/oder eines UV-Licht-Anteils die Lichtmenge aufsummiert wird.

13. Vorrichtung nach einem der vorhergehenden Ansprüche, wobei der Aktivitätssensor (2) einen Beschleunigungssensor und/oder einen Pulssensor aufweist.

14. Vorrichtung nach einem der vorhergehenden Ansprüche, wobei das Messgerät als am Handgelenk tragbares Armbandgerät ausgebildet ist.

15. Vorrichtung nach einem der Ansprüche 1 bis 13, wobei das Messgerät (1) als um den Hals tragbares Amulett, als Brille zur Sehhilfe ausgebildet ist, insbesondere in ein Brillengestell integriert ist, als Brosche, Ohrring, Fingerring, Gürtelschnalle oder elastisches Band ausgebildet ist.
